# EUROPEAN PATENT APPLICATION

(11) **EP 4 592 831 A1**
(43) Date of publication of application: **30.07.2025**
(21) Application number: 22969886.5
(22) Date of filing: 30.12.2022
(51) Int. Cl.: G06F 9/4401, C12M 1/36, C12M 1/34, G01N 35/00, G06F 21/74

(54) **METHOD AND APPARATUS FOR CONTROLLING CHIP LOADING, SEQUENCING SYSTEM, AND STORAGE MEDIUM**

(71) Applicant: GeneMind Biosciences Company Limited, Shenzhen, Guangdong 518023 (CN)
(72) Inventor: ZHOU, Yuan, Shenzhen, Guangdong 518023 (CN); SUN, Ruitao, Shenzhen, Guangdong 518023 (CN); CAI, Jinsen, Shenzhen, Guangdong 518023 (CN); LUO, Mingjie, Shenzhen, Guangdong 518023 (CN)
(74) Representative: Schiweck Weinzierl Koch Patentanwälte Partnerschaft mbB
(86) International application number: PCT/CN2022/144355
(87) International publication number: WO 2024/138746

(57) **Abstract**

A method and apparatus for controlling chip loading, a sequencing system, and a storage medium. When a current chip needs to be loaded, it is detected that another chip is executing a project, then if another chip is in an interference state and there is an unreached interruptible node, the execution situation of the project process of another chip is monitored; when the loading of the current chip has not been ended, it is detected that another chip has reached the interruptible node, and then another chip is caused to enter a safe mode until the loading of the current chip is ended; and the current chip executes a requested project, and another chip continues executing the interrupted project. Thus, another chip that is executing a project is caused to enter a safe mode at an appropriate time, other chips can be loaded flexibly, and multiple chips are loaded at different times and operate together, thereby improving the operation efficiency and industrial practicability of a detection system.

## Description

### TECHNICAL FIELD

The present disclosure relates to the field of automation control, particularly to the technical field of automated nucleic acid detection, and more particularly to a method for controlling chip loading, a corresponding apparatus, a sequencing system, and a computer-readable storage medium.

### BACKGROUND

The subject matter discussed in this section should not be regarded as prior art merely as a result of its mention in this section. Similarly, technical problems mentioned in this section or associated with the subject matter provided as background should not be considered as having been previously recognized in the prior art. The subject matter in this section only represents different methods, which may themselves correspond to specific embodiments of technical solutions in the claims.

For an automated platform that achieves the detection of a target biomolecule based on the detection of a surface of a solid substrate, processes generally include attaching the target biomolecule to the surface of the solid substrate, and using the automated platform to perform a series of biochemical reactions on the surface and acquire corresponding reaction signals, as well as to process and analyze the reaction signals to detect the target biomolecule.

Specifically, for a platform (sequencing platform) that achieves nucleic acid sequence determination by attaching a target nucleic acid molecule to a designated surface of a solid substrate and using a signal detection module to detect signals on the surface from or reflecting the target nucleic acid molecule, it is a feasible method to increase the area of the designated surface, such as increasing the number of designated surfaces or increasing the number of solid substrates to increase throughput (obtain a larger amount of sequencing data) in the case where the detection precision or resolution performance of the signal detection module is certain or difficult to improve.

However, in the case where the number of signal detection modules is certain, the signal acquisition performance of signal detection modules is certain, or it is expected that the volume and/or weight of a sequencing platform including such signal detection modules is not significantly increased, how to enable the sequencing platform to accommodate larger and/or more solid substrates with designated surfaces while improving the efficiency, overall operating efficiency, and/or flexibility of the sequencing platform for detecting these designated surfaces is a matter of concern.

### SUMMARY

According to a first aspect, an embodiment provides a method for controlling chip loading, including: step 101: acquiring an on-machine request, where the on-machine request includes an identifier of a current chip corresponding thereto and an identifier of a project requested to be executed on a machine; step 102: upon determining the presence of an additional chip that is executing a project and that the project executed by the additional chip belongs to a preset category, starting monitoring of a project process of the additional chip; step 103: upon determining that the project process of the additional chip has an interruptible node that has not been reached yet and the additional chip is currently in an interference-permissible state, starting loading of the current chip, where starting the loading of the current chip includes moving a carrying module for loading the current chip to an operable position; step 104: upon determining that the loading of the current chip has not been ended yet and the project process of the additional chip has reached the interruptible node, ending the monitoring of the additional chip and enabling the additional chip to enter a safe mode, where entering the safe mode includes interrupting the project process of the additional chip; and step 105: upon determining that the loading of the current chip has been ended, starting execution of the project requested by the current chip, and exiting the additional chip from the safe mode, where exiting from the safe mode includes resuming the interrupted project process of the additional chip.

The term "chip" as used herein refers to a solid substrate carrying a target biomolecule, such as a nucleic acid molecule, a protein, and the like. The term "on-machine" is generally understood to include processes of loading a chip onto a suitable machine, such as a suitable biomolecule detection device, apparatus, system, or platform, and then performing various operations or executing one or more projects on the machine to achieve specific detection of a target biomolecule in the chip. The term "project" is generally understood to refer to a process that has a definite start and end, and includes multiple interrelated parts or stages. A project has a corresponding project process, which is also referred to as a project operation process, and executing the project refers to entering the corresponding project process.

The project herein includes a process for achieving one or a set of specific objectives, or obtaining one or more specific results, on a chip or on a machine loaded with a chip under certain conditions, the process including one or more nodes and involving one or more states.

In certain examples, a stage, state, or node at which a project process is allowed to be interrupted, suspended, or paused for a certain period of time is referred to as an interruptible node.

In certain examples, a stage, state, or node at which a project process allows interference is referred to as an interference-permissible state.

In some examples, the term "interference-permissible state" includes one or more interruptible nodes. In other examples, the interference-permissible state and the interruptible node are independent of each other. In a certain specific example, after entering the interference-permissible state, the interruptible node may be reached after a specified duration and/or completion of a specified action or operation.

In certain examples, a project in which a chip undergoes a substantial change before and after execution of the project is categorized into a preset category, and the substantial change includes a significant difference in at least one of the following aspects of the chip compared with the chip before the execution of the project: the composition and structure of the chip, the physical and/or chemical properties of a designated location or position, such as a designated surface, of the chip, the structure of some or all of target biomolecules in the chip, etc.; the significant difference can be determined or identified by means of the naked eyes, conventional detection tools and/or means in the corresponding fields, etc.

In certain examples, the project belonging to the preset category includes executing the same series of operations or action instructions multiple times at different time points to complete the detection of the chip, such as sequencing by synthesis project, and more specifically, for example, a detection project of sequencing by synthesis based on chip imaging to achieve sequence determination of nucleic acid molecules carried on the chip. This detection project includes repeated multiple cycles of reactions, each cycle of reaction including executing a series of actions or operations to achieve base extension, signal acquisition, and removal of specific groups on the chip/nucleic acid molecule. In a certain example, each cycle of reaction includes one or more interference-permissible states and one or more interruptible nodes. In a certain example, the structure and/or physicochemical properties of the chip on which such a project is executed are generally changed significantly, making it difficult to reuse the chip for executing the project, or the cost required to restore the chip to the state or performance parameters before the execution of the project generally far exceeds the cost of preparing the chip.

According to a second aspect, an embodiment provides an apparatus for controlling chip loading, including: an on-machine request module, configured to acquire an on-machine request, where the on-machine request includes an identifier of a current chip corresponding thereto and an identifier of a project requested to be executed on a machine; a monitoring module, configured to, upon determining the presence of an additional chip that is executing a project and that the project executed by the additional chip belongs to a preset category, start monitoring of a project process of the additional chip; a loading start module, configured to, upon determining that the project process of the additional chip has an interruptible node that has not been reached yet and the additional chip is currently in an interference-permissible state, start loading of the current chip, where starting the loading of the current chip includes moving a carrying module for loading the current chip to an operable position; a safe mode module, configured to, upon determining that the loading of the current chip has not been ended yet and the project process of the additional chip has reached the interruptible node, end the monitoring of the additional chip and enable the additional chip to enter a safe mode, where entering the safe mode includes interrupting the project process of the additional chip; and an exit module, configured to, upon determining that the loading of the current chip has been ended, starting execution of the project requested by the current chip, and exit the additional chip from the safe mode, where exiting from the safe mode includes resuming the interrupted project process of the additional chip.

According to a third aspect, an embodiment provides a sequencing system, including the apparatus according to the above embodiment, or including one or more processors to execute the method according to the above embodiment.

According to a fourth aspect, an embodiment provides a computer-readable storage medium including a program, and executing the program can implement the method according to the embodiment of the present disclosure.

Based on the method for controlling chip loading and the corresponding apparatus, the sequencing system, and the computer-readable storage medium according to some embodiments described above, by identifying the project type of a chip that is executing a project and monitoring the project process (including monitoring whether the project being executed by the chip is in an interference-permissible state, whether the project reaches an interruptible node, etc.), relevant actions of starting the loading and/or loading an additional chip are performed, so as to realize multi-chip detection operation on the same machine without affecting the project executed by the current chip. The method loads other chips and/or takes out the current chip in a flexible manner at the proper time, enables multiple chips to be put on and off a machine at different time points and/or run different projects in parallel, realizes the rolling on-machine, off-machine, and/or parallel operation of the multiple chips, improves the operation efficiency and flexibility of a sequencing system, and meets the requirements or expectations of different application scenarios.

Based on the method for controlling chip loading and the corresponding apparatus, the sequencing system, and the computer-readable storage medium according to some embodiments described above, by monitoring whether a chip that is executing a project is in an interruptible node or an interference-permissible state or a safe mode, other chips can be loaded and/or the chip can be taken out in a flexible manner at the proper time, enabling multiple chips to be put on and off a machine at different time points and/or run different projects in parallel, realizing the rolling on-machine, off-machine, and/or parallel operation of the multiple chips, and thus improving the operation efficiency and flexibility of a sequencing system.

### BRIEF DESCRIPTION OF THE DRAWINGS

FIG. 1 is a schematic view of a project according to an embodiment of the present application;
FIG. 2 is a schematic structural view of a sequencing system according to an embodiment of the present application;
FIG. 3 is a schematic structural view of the sequencing system according to an embodiment of the present application;
FIG. 4 is a schematic flow chart of a method for controlling chip loading according to an embodiment of the present application;
FIG. 5 is a schematic flow chart of a method for controlling chip loading according to an embodiment of the present application;
FIG. 6 is a schematic flow chart of a method for controlling chip loading according to an embodiment of the present application;
FIG. 7 is a schematic flow chart of a method for controlling chip loading according to an embodiment of the present application;
FIG. 8 is a schematic flow chart of a method for controlling chip loading according to an embodiment of the present application;
FIG. 9 is a schematic flow chart of a method for controlling chip loading according to an embodiment of the present application;
FIG. 10 is a schematic flow chart of a method for controlling chip loading according to an embodiment of the present application;
FIG. 11 is a schematic flow chart of a method for controlling chip loading according to an embodiment of the present application;
FIG. 12 is a schematic flow chart of a method for controlling chip loading according to an embodiment of the present application;
FIG. 13 is a schematic flow chart of a method for controlling chip loading according to an embodiment of the present application;
FIG. 14 is a schematic flow chart of a method for controlling chip loading according to an embodiment of the present application;
FIG. 15 is a schematic flow chart of a method for controlling chip loading according to an embodiment of the present application;
FIG. 16 is a schematic structural view of an apparatus for controlling chip loading according to an embodiment of the present application.

### DETAILED DESCRIPTION

The present disclosure will be illustrated in further detail with reference to the following detailed description and drawings. Like elements in different examples have been given like numerals associated therewith. In the following embodiments, numerous specific details are given to provide a thorough understanding of the present application. However, those skilled in the art will recognize that some of the features may be omitted or substituted with other elements, materials, and methods in different instances. In some instances, certain operations related to the present application are not illustrated or described in this specification, and those skilled in the art can fully understand these related operations according to the description in the specification and the general knowledge in the art.

Furthermore, the illustrated features, operations, or characteristics in the specification may be combined in any suitable manner to give various embodiments. Also, the various procedures or actions in the description of the methods may be exchanged or adjusted in order, as will be predictable to those skilled in the art. Thus, the various orders in the specification and drawings are for the purpose of clearly illustrating certain embodiments only and are not intended to imply a necessary order unless otherwise indicated that a certain order is necessary. As used herein, the singular form "a", "an", "the", or the like includes its plural referent, that is, the singular form indicates that the number is one or more.

As used herein, the term "sequencing" refers to sequence determination, and is used interchangeably with "nucleic acid sequencing" and "gene sequencing" to refer to the determination of base order in nucleic acid sequences, including sequencing by synthesis (SBS) and/or sequencing by ligation (SBL), including DNA sequencing and/or RNA sequencing, and including double-end sequencing, single-end sequencing, paired-end sequencing, and/or the like (the double-end sequencing or paired-end sequencing may refer to the reading of any two segments or portions of the same nucleic acid molecule that are not completely overlapping).

The sequencing includes the process of binding nucleotides (including nucleotide analogs) to a template and acquiring the corresponding reaction signals. Some sequencing platforms where the binding of nucleotides to the template and the acquisition of corresponding reaction signals are conducted asynchronously/in real-time generally involve multiple cycles of sequencing to determine the order of multiple nucleotides/bases on the template. A "cycle of sequencing", also referred to as a "sequencing cycle", may be defined as one base extension of one or more types of nucleotides/bases, and in other words, as the determination process of the base type at a designated position on at least a part of the template.

As used herein, the term "chip" may be any solid support useful for immobilizing nucleic acid sequences, such as nylon membranes, glass slides, plastics, silicon wafers, magnetic beads, and the like, and may sometimes be referred to as a solid substrate, reactor, or flow cell. The chip may carry a target biomolecule, such as a nucleic acid molecule, a protein, and the like.

The automation of all or part of the steps or processes of sequencing may be achieved by designing an automated detection platform, such as a sequencing system platform. As used herein, an automated sequencing platform is also referred to as a sequencing platform or a sequencing system, and includes a fluid path module, a signal acquisition module, and the like, and target nucleic acid molecules are generally chemically or physically attached to a designated surface of a solid substrate (chip), so that the sequencing platform may perform automated detection on the chip and output detection data or results of the target molecules thereon, including sequence determination of the target nucleic acid molecules thereon. One or more projects including operation interfaces of corresponding projects may be preset on the sequencing platform, and a user is supported to select a specific project and execute the process of the project to complete the project detection, or the project may be established and the corresponding project process may be executed according to the received user instruction to complete the project detection.

In some embodiments, for a platform that achieves sequencing based on a polymerization reaction, a base extension reaction system includes reaction substrate nucleotides (including nucleotide analogs), a polymerase, and a template (a target nucleic acid molecule); a predetermined sequence (a sequencing primer) is bound to the template, and on the basis of the base pairing principle and the rationale of the polymerization reaction, the reaction substrate is controllably connected to the 3' end of the sequencing primer under the catalysis of the polymerase to achieve the pairing with the base at a corresponding position of the template (the complementary strand of the template is extended by one base). Generally, such sequencing includes multiple repeated base extension reactions (repeats); the base extension reaction includes the process of binding the nucleotides to the template and acquiring corresponding reaction signals, and such a repeated reaction may also be referred to as a cycle of reaction. For example, four types of nucleotides are sequentially added to the reaction system to each perform base extension and corresponding acquisition of reaction signals, such that base calling at any designated position of the template includes four base extensions and the corresponding signal acquisitions; for another example, four types of nucleotides are added to the reaction system in any combinations (such as in pairs or in one-three combination), the two combinations each perform base extension and corresponding acquisition of reaction signals, such that base calling at any designated position of the template includes two base extensions and the corresponding signal acquisitions; for yet another example, four types of nucleotides are added simultaneously to the reaction system for base extension and acquisition of reaction signals, such that base calling at any designated position of the template includes one base extension and the corresponding signal acquisitions.

The sequencing mode may select single-end sequencing or double-end sequencing. The obtained sequencing results/data (i.e., read fragments) are referred to as reads, and the length of a read is referred to as read length. Sequencing may be performed through an automated sequencing platform, and the sequencing platform may be selected from, but is not limited to, the Hiseq/Miseq/Nextseq/Novaseq sequencing platform (Illumina), the Ion Torrent platform (Thermo Fisher), the BGISEQ and MGISEQ/DNBSEQ platforms (MGI), single-molecule sequencing platforms (PacBio), and the like.

The mainstream high-throughput sequencing platform usually performs amplification on target nucleic acid molecules in a chip on a machine to obtain a clonal cluster, or loads the amplified clonal cluster or ball, such as a DNA nanoball, to a designated position on a designated surface of the chip, and then performs automated sequencing on the amplification product (clonal cluster or ball). The term "sequencing" or "sequencing project" as used herein sometimes includes the process of amplifying target molecules on the surface of a chip, as will be understood by those of ordinary skill in the art through the context.

The term "target nucleic acid molecule" in the chip is generally referred to as a library and is usually the processed target nucleic acid molecule. For example, the library is prepared by connecting a predetermined sequence (linker) to the end of a target fragment (also referred to as an insert fragment) from a sample, and the library is then hybridized/captured by using a chip having a primer or probe (oligonucleotide strand) immobilized on the surface of the chip, which can be complementarily hybridized with at least a part of the linker, such that the library is attached to the designated surface of the chip by complementary binding to the primer or probe.

The amplification of target molecules/libraries may be performed in the liquid phase and/or solid phase, and may be achieved by isothermal amplification or non-isothermal amplification methods, which is not limited in the present application. For example, the target molecule may be amplified on the surface of a substrate by using bridge amplification (bridge PCR; see the disclosure of US20050100900A1) or template-walking amplification (see Zhaochun Ma et al., PNAS, 110(35):14320-14323, Aug. 27, 2013). For example, the target molecule is amplified in a solution, and then the amplification product is attached to the designated position on the surface. For example, the target molecule is cloned by using rolling circle amplification in a solution, and the amplified target molecule (also referred to as a DNA nanoball (DNB)) is loaded on the surface.

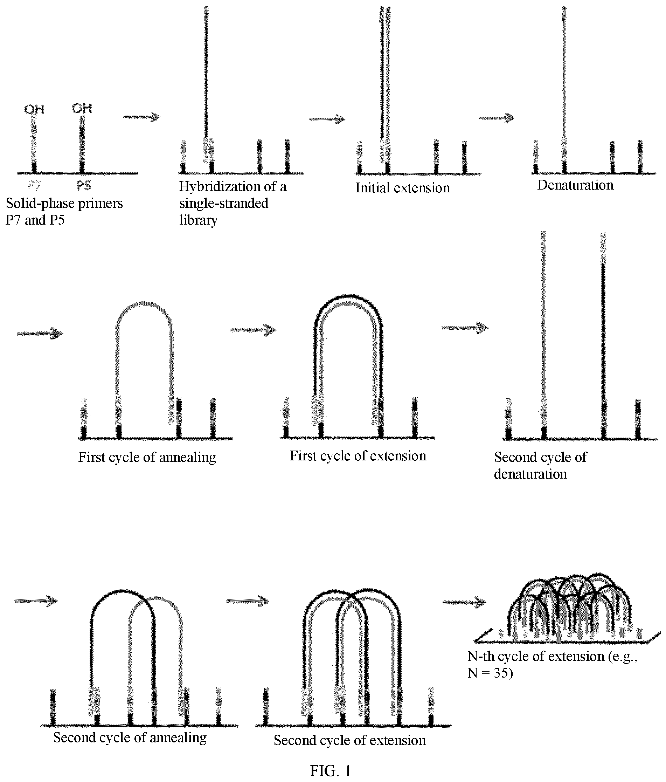

FIG. 1 illustrates a method for bridge amplification used by the ILLUMINA sequencing platform to obtain a clonal cluster of target molecules on a surface, and this process briefly includes: obtaining a single-stranded library by melting; hybridizing the single-stranded library with a substrate having two primers/probes immobilized on the surface thereof, which may be referred to as a P7 solid-phase primer and a P5 solid-phase primer and may be hybridized with 3' ends of the two complementary strands of the library, respectively; extending the P7 or P5 solid-phase primer to synthesize the complementary strand of the single-stranded library; obtaining a new single-stranded template (complementary strand) by denaturation; performing annealing to allow the complementary strand to hybridize with the P5/P7 solid-phase primer; and extending the P5/P7 solid-phase primer to synthesize a new complementary strand. As such, by one or more runs (cycles) of denaturation - annealing - extension, amplification products (a clonal cluster) are obtained. Sequencing, such as single-end sequencing or double-end sequencing, is then performed on the amplification products to obtain a sequencing result. It can be seen that the process includes multiple steps/reactions, involving the input and replacement/output of various reagents, and/or the regulation of the temperature of the solution system. In a certain example, an interruptible node and/or an interference-permissible state may be set during operations between two steps that may involve introducing a solution to remove a specific component in the previous step (e.g., a component that may affect the next step) or replacing the solution system to better proceed with the next step, after "hybridizing the single-stranded library" and before "initial extension" and so on. In another example, considering that the amplification process shown in FIG. 1 takes a relatively short time and the associated steps/reactions involving enzymatic catalysis are generally required to be performed in a solution environment having a suitable pH range and/or temperature, the reaction efficiency and/or product yield is relatively susceptible to various factors such as time, temperature, etc., and the amplification process is generally less suitable for being interfered with or interrupted in the middle or for a longer period of time. Therefore, no interference-permissible state and/or interruptible node is set during this period.

In certain examples, after the amplification is completed, for example, after "N-th cycle of extension" shown in FIG. 1 and before sequencing the amplification products, the steps of strand cleaving (e.g., enabling P5 to have an enzyme-cleavable site), linearization, blocking (e.g., blocking the 3' end of P5), hybridizing the sequencing primer to the single-stranded amplification product/template, and the like are also included. In order to improve the reaction efficiency of each step or to avoid being affected by the previous step, a step of introducing a buffer to remove or replace the previous reaction system or solution is often provided between the two steps before and after these steps, and such a solution replacement operation similar to the cleaning and washing functions usually does not involve substantial processing/biochemical reactions, and an interruptible node and/or an interference-permissible state may be generally set either when entering this step or during this step.

Sequencing is performed on the amplification products, for example, in certain examples, multicolor SBS is performed on the amplification products. Specifically, four types of nucleotides (dNTPs) correspond to different signals, for example, carrying different fluorescent labels F1, F2, F3, and F4, and the luminescence wavelengths of any two of the fluorescent labels F1, F2, F3, and F4 do not completely overlap, or the four types of nucleotides sequentially carry fluorescent labels P1, P2, a mixture of P1 and P2, and no any fluorescent label. The "nucleotides" as used herein, also commonly referred to as "engineered nucleotides", "nucleotide analogs", or "reversible terminators", are nucleotide analogs carrying detectable labels (such as fluorescent labels) and blocking groups. One cycle of four-color sequencing includes adding four types of dNTPs for the single-base extension reaction, exciting the fluorescent labels to emit light after the extension reaction, and acquiring multiple images of the corresponding dNTPs/fluorescent labels in each field of view (FOV).

In some specific embodiments, SBS is performed by using reversible terminators on the basis of chip detection, and a cycle of reaction includes:
(1) performing single-base extension, including introducing a solution suitable for the base extension into a chip, and binding dNTPs to a template under the catalysis of a polymerase at a suitable temperature, where the dNTPs carry fluorescent labels and blocking groups capable of inhibiting the binding of the nucleotides to the next position of the template;
(2) performing signal acquisition, such as photographing, including introducing a solution suitable for fluorescence detection into the chip, exciting the fluorescent labels to emit light, and using a camera to acquire corresponding luminescent signals; and
(3) performing cleavage, including introducing a solution capable of removing a specific group, and cleaving the fluorescent labels and the blocking groups on the dNTPs bound to the template.

As such, steps (1)-(3) are repeated multiple times.

Sequencing may include multiple cycles of reactions, such as N cycles of reactions. In some embodiments, N is greater than 50. Therefore, the base types at multiple positions of the target molecule may be determined based on the detection data of the multiple cycles of reactions, that is, the determination (base calling) of at least a part of the sequence of the target molecule is achieved, and reads are obtained.

Above, reading the sequence at one end of the target molecule may be referred to as single-read sequencing. If the sequence at the other end of the target molecule is intended to be read, it may further include: a. removing the extended strand; b. performing annealing-extension to synthesize the complementary strand of the template; and c. adding a sequencing primer/hybridizing the sequencing primer with the complementary strand of the template, and then performing multiple cycles of reactions similar to the above to obtain the sequence (reads2) at the other end of the target molecule. In certain examples, in order to prevent the solution/reagent in the previous step from having undesirable effects on the reaction in the next step, operations of removing the specific solution/reagent component in the previous step or replacing the solution system, similar to the cleaning function, may be involved between a and b, or between b and c, and an interruptible node may be set either when entering such operations or during the period of performing such operations. In other examples, similar to the amplification in the above examples, no interference-permissible state and/or interruptible node is set during this period.

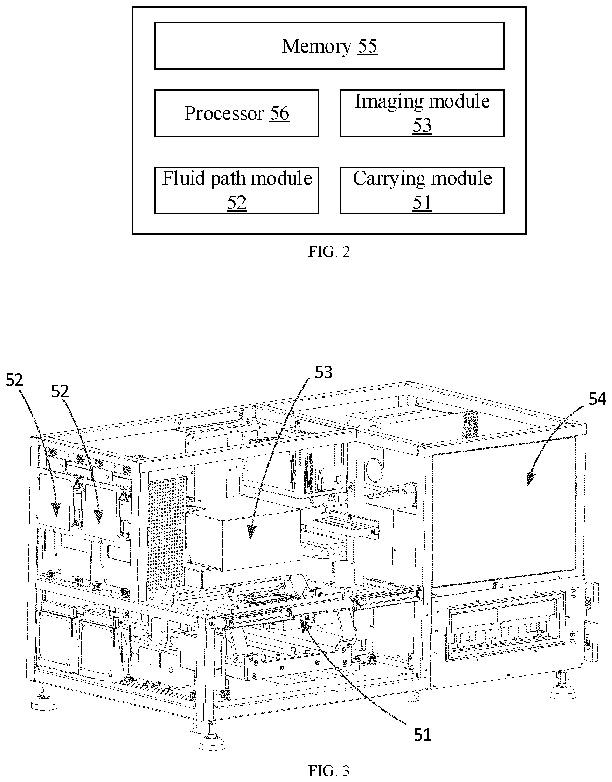

The method according to any one of the embodiments of the present application can be applied to a platform for achieving biomacromolecule detection based on chip detection, such as a sequencing system based on chip imaging to determine the sequence of nucleic acid molecules therein. Referring to FIG. 2, in some embodiments, the sequencing system may include a carrying module 51, a fluid path module 52, and an imaging module 53; in some embodiments, the sequencing system may further include one or more memories 55, and one or more processors 56, which are described in detail below.

In some embodiments, the carrying module 51 is configured to carry a chip, including loading and moving the chip, as well as setting the corresponding reaction environment, for example, thereby enabling the chip to have a reaction environment suitable for enzymatic biochemical reactions. In some embodiments, the reaction environment includes temperature, solution environment, etc. The carrying module 51 includes a connected carrier, a temperature control structure, and a motion structure. The temperature control structure includes, for example, a Peltier cooling plate, etc., which can heat or cool the chip placed on the carrier, and the motion structure can drive the carrier and/or the temperature control structure to move in a horizontal and/or vertical direction to the optical axis.

In some embodiments, the fluid path module 52 is configured to control the addition and extraction of reaction reagents during sequencing. In some embodiments, the fluid path module 52 is connected to the carrying module 51, and includes a pipeline, a valve body, and a power unit (e.g., a pump), etc. for controlling reagents to enter and exit the chip.

In some embodiments, the imaging module 53 is configured to photograph the chip and acquire sequencing data for subsequent analysis. In some embodiments, the imaging module 53 is connected to the carrying module 51 and the processor 56 for acquiring image signals from the designated area of the chip. The imaging module 53 includes an optical axis, an objective lens, and a camera (e.g., CCD, TDI-CCD, CMOS, or TDI-CMOS, etc.). In combination with the carrying module 51, the imaging module 53 can intermittently or continuously acquire signals from multiple fields of view of the designated surface of the chip.

Therefore, the carrying module 51 loads the chip and the nucleic acid molecules in the chip are placed under the conditions suitable for the designated reaction; the fluid path module 52 controls the input or output of solutions to or from the chip to make the nucleic acid molecules perform the designated reaction, such as the incorporation of one or more nucleotides into the nucleic acid molecules under the catalysis of a DNA polymerase; the imaging module 53 acquires the images on the designated field of view of the chip to obtain the image signals of the nucleic acid molecules after the designated reaction; and these image signals are analyzed to obtain sequencing data and determine the sequence of the nucleic acid molecules.

In certain specific examples, sequencing, e.g., sequencing by synthesis, includes multiple repeated reactions, and one or more nucleotides are incorporated into the target nucleic acid molecule through one repeated reaction, that is, at least a part of the target nucleic acid molecule undergoes a single-base extension reaction. One repeated reaction may be referred to as one cycle of reaction. In some examples, the reagents or solutions are input to or output from the chip, which may be implemented using the fluid path module 52; for example, the base extension involves enzymatic catalysis, which may be performed at a temperature higher than room temperature to facilitate the improvement of the efficiency of enzyme-catalyzed polymerization reaction, and the chip may be heated by the carrying module 51 including the temperature control structure; then, during information acquisition or photographing, the fluorescence detection solutions are introduced using the fluid path module 52, and the temperature of the chip is optionally adjusted in combination with the carrying module 51, such that the chip is in an environment suitable for fluorescence imaging; the chip after the base extension reaction is excited by the imaging module 53, the luminescence signals are acquired to obtain images, and in this process, the chip is moved by the carrying module 51 and/or the objective lens of the imaging module 53 is moved, such that images of multiple fields of view on the chip are acquired.

Referring to FIG. 3, in some embodiments, the sequencing system may further include a display 54. The display 54 is configured to display an interaction interface, and in some embodiments, the display 54 is a touch screen. In some embodiments, the touch screen 54 may display one or more pages, such as a graphical user interface. The number of pages is equal to the number of chips that the sequencing system may simultaneously carry.

In some embodiments, the sequencing system may simultaneously carry multiple chips, e.g., 2 chips. In the case that the sequencing system may simultaneously carry multiple chips, the number of the fluid path modules 52 may be the same as the number of chips that the sequencing system can simultaneously carry at most. For example, in the case that the sequencing system may simultaneously carry 2 chips, the number of the fluid path modules 52 is also 2.

In an embodiment where the sequencing system may simultaneously carry multiple chips and the multiple chips involve a shared module or structure, for example, the carrying module 51 is provided with multiple positions where the chips can be loaded. When the carrying module 51 currently carries one chip (e.g., chip A) and the sequencing system is executing a project on chip A, if an additional chip (e.g., chip B) is currently intended to be loaded, the carrying module 51 may be moved to an operable position, e.g., to a position where a user may place or load chip B for operation, at an appropriate time. It can be understood that, since the carrying module 51 carries chip A at the same time, chip A moves along with the carrying module 51 during the movement. After moving the carrying module 51 to the operable position, the user places chip B onto the carrying module 51 to load chip B, and after completing the loading of chip B, the project of chip A may be continued, and the execution of the project of chip B is started. In some examples, the appropriate time refers to a time point when the loading of chip B is started without affecting the project that chip A is executing.

Therefore, in some embodiments of the present application, when it is determined that the additional chip is already executing the project in the sequencing system and it is determined that the additional chip is currently in an interference-permissible state, the current chip may be started to be loaded by the carrying module 51; when it is determined that the project executed by the additional chip reaches an interruptible node and the loading of the current chip has not been ended, the project process of the additional chip may be paused and the additional chip enters a safe mode until the loading of the current chip is ended; after the loading of the current chip is ended, the current chip may execute the requested project, and the additional chip may continue to execute the paused project.

In other embodiments, multiple chips execute their respective projects in the sequencing system, and the unloading of the current chip is started when the project of the additional chip is executing and its project process is determined to be in an interference-permissible state; when it is determined that the project executed by the additional chip reaches an interruptible node and the unloading of the current chip has not been ended, the project process of the additional chip may be paused and the additional chip enters a safe mode until the unloading of the current chip is ended; after the unloading of the current chip is ended, the additional chip may continue to execute the paused project.

Therefore, the multiple chips can be loaded into or unloaded from the sequencing system at different time points, and can independently execute respective projects in parallel at the same time during sequencing, which is convenient to efficiently and flexibly obtain results of multiple detection projects of the multiple chips, and is suitable for various applications, in particular to applications where the detection time is limited, but sometimes the number of samples is large or small, or the number of samples is often difficult to reach the full loading number of the chips/the sequencing system in a short period of time, such as pathogen detection and the like.

In some embodiments, the sequencing system may support the loading of multiple chips and enable the multiple chips to execute respective projects independently or interdependently. In some embodiments, the carrying module 51 is shared by multiple chips. For example, the two chips described above may be loaded simultaneously, or may be loaded onto the shared carrying module 51 in a time-sharing manner. In some embodiments, the imaging module 53 is shared by multiple chips. In some embodiments, the number of the fluid path modules 52 corresponds to the number of chips that the carrying module 51 can carry, such that in an application scenario where multiple chips need to be simultaneously operated, the inlet and outlet of solutions in each chip can be independently controlled, more operation modes such as single-chip detection, simultaneous detection of multiple chips, time-sharing detection of multiple chips, and the like can be provided to meet the requirements of different applications, and thus the sequencing platform has stronger flexibility and higher operation efficiency.

The term "project" has a corresponding project process, which is also referred to as a project operation process, and executing the project refers to entering the corresponding project process. Each project includes one or more associated steps, operations, or nodes including the execution order among the steps, operations, or nodes and including multiple stages such as starting, executing, performing control, ending, etc. **In** the case that a project process is interfered with in a certain stage, step, or node (e.g., a chip that is executing the project is accidentally moved, an ongoing operation action is paused, etc.), if the result of the stage or step or the final result of the project is not affected thereby, or the effect is negligible, or the effect is within an acceptable range, the project process of the chip in this stage, step, or node may be referred to as being in an interference-permissible state, or the chip executing the project in this stage, step, or node is referred to as a chip in an interference-permissible state. Conversely, for the project process is interfered with in other stages, steps, or nodes, if the obtained result of the step or stage or the final result of the project is significantly affected thereby, or if the effect is non-negligible, then the project process of the chip in these stages, steps, or nodes may be referred to as being in an interference-impermissible state, or the chip executing the project in these stages, steps, or nodes may be referred to as a chip in an interference-impermissible state. In a certain example, it is determined by monitoring that the chip is executing a sequencing project and is in a stage (e.g., the imaging module 53 is in a working state and/or the carrying module 51 is in a motion state according to the above examples) for signal acquisition/photographing, and the project process of the chip is determined to be in an interference-impermissible state. In a certain example, it is determined by monitoring that the chip is executing a sequencing project and is between, e.g., the base extension stage and the signal acquisition/photographing stage according to the above examples (the project process of the chip can be determined between these two stages/steps by monitoring the end of the base extension stage and/or starting the fluid path module 52, e.g., starting the pump to pump a solution suitable for fluorescence detection to the chip, etc.; whether the base extension stage is ended can be monitored and determined as follows, e.g., when the fluid path module 52 is started to introduce the solution suitable for base extension into the chip, timing is started, and if the timing has reached a specified duration, e.g., three minutes, the end of the base extension step can be determined), and the project process of the chip is determined to be in an interference-permissible state.

Therefore, by determining whether the project process executed by chip A is in an interference-permissible state or not, an appropriate time for loading or unloading chip B can be determined, so as to avoid or reduce the effect of loading or unloading chip B on chip A that is currently executing the project. When the project process corresponding to the project being executed by chip A is in an interference-permissible state, loading or unloading of chip B may be started, for example, the carrying module 51 is moved to the operable position to start the loading or unloading of chip B.

In summary, it is relative that the project process of the chip is in an interference-permissible state and in an interference-impermissible state. The interference-permissible state is used to indicate that the stage, step, or node in which the project process of the chip may be interfered with, even interrupted, suspended, or paused, and the interference-permissible state includes one or more interruptible nodes where the chip may execute instructions or actions outside the project process, e.g., moving the chip to the operable position along with the movement of the carrying module 51, which will not therefore adversely affect the accuracy or amount of the result of the step being executed by the chip or the final result of the project, such as the detected sequencing data. The interference-impermissible state is used to indicate that the stage, step, or node in which the project process of the chip may not be interfered with (including not being interrupted, suspended, or paused, or preferably not being interfered with, interrupted, or paused); during this period, the chip is not interfered with by actions or operations outside the project process, is not moved in position, and is not moved along with the movement of the carrying module 51, the temperature is not suddenly changed, the operation or action being executed by the chip is not suddenly interrupted, etc., so as to avoid or reduce adverse effects on the accuracy of the result of the ongoing step or on the final result of the project, such as the detected sequencing data.

In certain examples, the interference-permissible state does not include an interruptible node, with specific time intervals, step intervals, and/or action instruction intervals between the interruptible node and the interference-permissible state in the same project process. In a certain example, the operable position is located inside the sequencing system, for example, a structure separated from the outside, such as a structure similar to a hatch door, is also arranged at the operable position. After the loading of the current chip is started (including the carrying module 51 has moved to the operable position), completing the loading of the current chip further involves a series of operations such as opening the hatch door manually or non-manually (e.g., by a mechanical arm or an electronic command), placing the current chip in a designated position of the carrying module 51, closing the hatch door, and the like. Even during this period, the position of the additional chip needs to be adjusted or the additional chip needs to be reloaded due to an operation error or an accidental touch of the additional chip, and opening the hatch door is set as an interruptible node that the project process of the additional chip reaches, such that the monitoring of the additional chip may be ended and/or the additional chip enters a safe mode.

It can be understood that, in this example, when the project process is in the interruptible node, the project process must be in the interference-permissible state, whereas when the project process is in the interference-permissible state, the project process is not necessarily in the interruptible node.

In some embodiments, the interruptible node is preset, and may be set in the project execution process, or may be set after the execution of a certain step in the project is ended. As described above, the project process refers to one or more execution steps corresponding to a project and the execution order among the execution steps. In principle, an interruptible node may be set between the current execution step and the next execution step, as long as a certain execution step is suspended or interrupted for a certain time without affecting the execution and implementation of the subsequent steps.

Both the interference-permissible state and the interruptible node may be set by experimentation, testing, and/or experience.

For example, the reaction efficiency is relatively low, and the requirements for performing reactions are relatively high (e.g., the reaction requirements include a specified pH range and/or temperature condition), and/or there are steps corresponding to components/reagents with relatively active physical or chemical properties, which easily affect the surface performance and/or easily affect target molecules. Since the efficiency of such steps/reactions per se is not high/relatively uncontrolled, or the cost of resuming the steps/reactions after the interruption is relatively high, and uncertain factors affecting the implementation of the steps/reactions are increased, the state before the steps/nodes is generally not recommended to be set or determined as an "interference-permissible state" (i.e., belonging to an "interference-impermissible state"). For another example, before executing the next step immediately after a certain node, the solution in the current step (or the solution in the current step is in a buffer that does not affect the subsequent reactions) is waited for a period of time (e.g., 5 minutes, 10 minutes, or longer), and then the subsequent steps are performed. Subsequently, whether there is a substantial difference between the obtained project detection result and the detection result obtained under a standard process (i.e., the solution does not undergo the above waiting time) is determined, and if there is no substantial difference, it indicates that the next step immediately after the above node is in an interference-permissible state, and the above node is also an interruptible node.

In some embodiments, the operation of moving the carrying module 51 during the photographing in the project process will affect the photographing result, and thus the project process is in an interference-impermissible state during the photographing;
in some embodiments, the operation of moving the carrying module 51 during the biochemical reaction in the project process has little effect on the final result, and thus the project process is in an interference-permissible state during the biochemical reaction.

As described above, the interruptible node may or may not be set in the interference-permissible state; however, the interruptible node is not present in the interference-impermissible state.

In some embodiments, if no interruptible node is set in the interference-permissible state, the sequencing system may set a node that is converted from the interference-permissible state to the interference-impermissible state as the interruptible node by default.

In some embodiments, the interruptible node and the interference-permissible state may be set independently or in association; in some embodiments, the interruptible node may be set before steps or nodes that can be performed again after a certain time.

The above is a description of the interference-permissible state and the interruptible node, and whether the project belongs to the preset category is described below.

In some embodiments of the present application, projects are classified according to whether the projects themselves (or the project processes) may be interfered with, interrupted, or suspended at any time. For example, some projects are classified into preset categories, and if the projects belong to the preset categories, it indicates that the corresponding project processes cannot be interfered with or interrupted at any time, but the project processes can be interfered with, interrupted, or suspended in some steps or between steps; when the projects do not belong to the preset categories, the project processes may be interfered with, interrupted, or suspended at any time. In other words, when a project process is in the interference-impermissible state in some stages, steps, or nodes, the corresponding project belongs to the preset category, and thus the project belonging to the preset category is a project having the interference-impermissible state.

It can be seen that the preset category is used to indicate that the corresponding project cannot be interfered with, interrupted, or suspended at any time during the execution process (i.e., the project process). As described above, this is because the corresponding project process may be in the interference-impermissible state in some stages, steps, or nodes.

In some embodiments, for the project belonging to the preset category, the project process thereof has more than two interruptible nodes; in some embodiments, the interruptible node is a preset node before the interference-impermissible state, or the interruptible node defaults to a previous node that enters the interference-impermissible state.

In some embodiments, the project belonging to the preset category is a project having the interference-impermissible state; in some embodiments, these projects have interference-impermissible states, including a photographing state.

In some embodiments, for the project belonging to the preset category, the project process thereof includes some steps, nodes, or time periods in the interference-permissible state and some steps, nodes, or time periods in the interference-impermissible state, that is, the project belonging to the preset category may temporally exhibit conversion from the interference-permissible state to the interference-impermissible state, and/or conversion from the interference-impermissible state to the interference-permissible state. That is, in some embodiments, for the project belonging to the preset category, the project process thereof may include multiple interference-impermissible states and multiple interference-permissible states, and the interference-impermissible states and the interference-permissible states are alternated, such that an interruptible node may be set at a node before each interference-impermissible state, that is, an interruptible node may be set at a node at which the interference-permissible state ends; if the interruptible node is not set, the node before the interference-impermissible state is set as the interruptible node by default. As such, the setting of interruptible nodes may be more flexible.

In some embodiments, the sequencing project belongs to the preset category, and the washing project does not belong to the preset category.

In some embodiments, the sequencing project may include, in sequence, the following execution steps: loading a chip, executing hybridization amplification, executing multiple biochemical reactions and photographing (which may include executing a first biochemical reaction, executing a first photographing, executing a second biochemical reaction, executing a second photographing until executing n-th biochemical reaction and executing n-th photographing, where n is an integer greater than or equal to 1), and executing cleaning; during the execution of the sequencing project, the photographing and other steps cannot be interrupted, otherwise, the sequencing process is not standardized, and the sequencing result is inaccurate.

In some embodiments, the cleaning project may include, in sequence, the following execution steps: loading a chip and executing cleaning.

The above is a description of whether the project belongs to the preset category. How to implement on-machine management of the sequencing chip is described below in combination with the interference-permissible state, the interruptible node, and whether the project belongs to the preset category.

Some embodiments of the present application provide a method for controlling chip loading, which may be applied to the sequencing system disclosed herein, and the sequencing chip may be a chip involved in the sequencing system; in such an embodiment, the memory 55 is configured to store data and a program, and the processor 56 is configured to, when executing the program stored in the memory 55, implement the method for controlling chip loading according to any one of the embodiments of the present application.

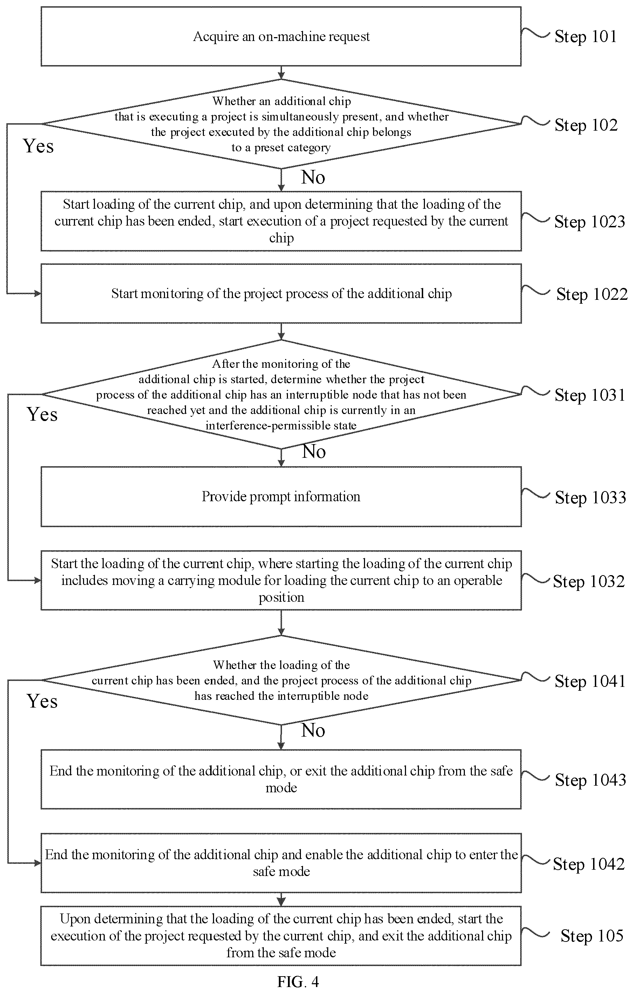

Referring to FIG. 4, the method for controlling chip loading according to some embodiments includes the following steps:
Step 101: acquiring an on-machine request.

The acquired on-machine request may be an instruction in various forms. The on-machine request may be a touch instruction received by the sequencing system at a designated position in a preset display interface, for example, a long press of a load button in a chip placement interface displayed in the touch screen 54 of the sequencing system starts a chip loading operation, or a click on a "load" option in a loading interface starts the chip loading operation. The on-machine request may also be a voice instruction, for example, the sequencing system receives a voice message such as "chip loading", "start loading", "start loading a new chip", and the like input by voice, and then starts the chip loading operation. The on-machine request may also be a preset air gesture instruction, for example, when the sequencing system receives a preset slide gesture, or when a user draws a preset shape in the air, the chip loading operation is started. It can be understood that the on-machine request is not limited to the above forms, and may be in other predefined forms. The received on-machine request above may be in various forms, and the user can conveniently select the on-machine request according to actual needs, such that the chip loading operation is started.

In some embodiments, the on-machine request includes an identifier of a current chip corresponding thereto and an identifier of a project requested to be executed on a machine. The on-machine request is used for requesting to execute an on-machine operation on the current chip and to execute the project indicated by the identifier of the project.

The identifier of the chip may be used for distinguishing multiple different chips, for example, for distinguishing multiple chips in different states such as standby, on a machine, on-machine running, off-machine/end of running, or the like, for distinguishing multiple chips executing different projects or executing the same project but in different steps, stages, or nodes, and the like. It can be understood that input of the identifier of the current chip may distinguish the current chip from other chips, including indicating the position of the current chip, the executed project, the specific stage of the executed project, or the like, and acquiring the on-machine request includes reading or inputting the identifier of the current chip, so as to trace or track the current chip, query the state of the current chip, and distinguish the current chip from other chips, including distinguishing the states, positions, and the like of multiple current chips.

In some embodiments, the identifier of the chip may be represented by a number, a letter, a pattern, a QR code, a barcode, or a combination of symbols in various forms, which is not limited in the present application, so as to facilitate reading the identifier of the chip by using a reader, a mobile phone, a computer, or other devices or modules (such as a nucleic acid sequencing platform) having identifier recognition and scanning functions and directly or indirectly connected to the machine.

In some embodiments, there may be multiple projects involved in the on-machine request, each project having a corresponding identifier.

In some embodiments, the identifier of the project may indicate the type of the project, which is used for distinguishing different types of projects. The identifier of the project may be a combination of one or more forms of a number form, a letter form, a pattern form, and the like, for example, the identifier of the sequencing project is project 1, and the identifier of the non-sequencing project (e.g., washing project before or after the machine runs) is project 2. The identifier of the project may be used to determine whether the project belongs to a designated category. Step 102: upon determining the presence of an additional chip that is executing a project and that the project executed by the additional chip belongs to a preset category, starting monitoring of a project process of the additional chip.

When the project executed by the additional chip belongs to the preset category, the project generally cannot be interfered with, interrupted, or suspended at any time, and thus the monitoring of the project process of the additional chip is started to determine whether the interference-permissible state or the interruptible node is present currently to find an appropriate time for performing or starting the loading of the current chip.

Therefore, in some embodiments, step 102 may include steps 1021-1023 below:
Step 1021: determining whether an additional chip that is executing a project is present, and whether the project executed by the additional chip belongs to a preset category.

After acquiring the on-machine request, the sequencing system determines whether the additional chip that is executing the project is present and the category of the project executed by the additional chip, that is, whether an additional chip that is executing a project and where the executed project cannot be interrupted at any time is present in the sequencing system is determined. In some embodiments, whether the additional chip that is executing the project is present may be first determined. If the additional chip that is executing the project is present, whether the project executed by the additional chip belongs to the preset category is then determined. If it is determined that the additional chip that is executing the project is present and the project executed by the additional chip belongs to the preset category, step 1022 is executed to start the monitoring of the project process of the additional chip; conversely, for example, if it is determined that the additional chip that is executing the project is not present, or the additional chip that is executing the project is present but the project executed by the additional chip does not belong to the preset category, step 1023 may be executed to start the loading of the current chip, and upon determining that the loading of the current chip has been ended, the execution of the project requested by the current chip is started.

It can be understood that, through the above determination results, when it is determined that the loading of the current chip will not affect other chips or other projects executed by other chips, the loading of the current chip may be started, so as to execute the project requested by the current chip (i.e., execute step 1023). If it is detected that the project being executed by the additional chip belongs to the preset category, the chip loading operation cannot immediately be performed on the current chip, and thus it is necessary to execute step 1022 to start the monitoring of the project process of the additional chip, and find an appropriate time, node, or stage to load the above current chip by monitoring whether the project process is currently in an interference-permissible state and interruptible node.

Step 103: upon determining that the project process of the additional chip has an interruptible node that has not been reached yet and the additional chip is currently in an interference-permissible state, starting loading of the current chip, where starting the loading of the current chip includes moving a carrying module for loading the current chip to an operable position.

In some embodiments, step 103 may include steps 1031-1033 below: step 1031: after the monitoring of the additional chip is started, determining whether the project process of the additional chip has an interruptible node that has not been reached yet and the additional chip is currently in an interference-permissible state. If yes, step 1032 is executed. If no, step 1033 is executed.

Step 1032: starting loading of the current chip, where starting the loading of the current chip includes moving a carrying module for loading the current chip to an operable position, and executing step 104.

Step 1033: providing prompt information. In some embodiments, the prompt information includes an instruction indicating that the current chip needs to wait. In some embodiments, the prompt information further includes a waiting time required for the current chip; in some embodiments, the required waiting time may be determined based on the time required for the additional chip to convert to the interference-permissible state. It can be seen that, in step 1031, when determining whether the project process of the additional chip has an interruptible node that has not been reached yet and the additional chip is currently in an interference-permissible state, if the determination result is "no", it indicates that the loading of the current chip will interfere with the project process being executed by the additional chip, the current chip cannot be immediately loaded, and the sequencing system may provide prompt information to prompt the current chip to wait for an instruction that the loading is available.

Step 104: upon determining that the loading of the current chip has not been ended yet and the project process of the additional chip has reached the interruptible node, ending the monitoring of the additional chip and enabling the additional chip to enter a safe mode. In some embodiments, entering the safe mode includes interrupting the project process of the additional chip.

It can be seen that, since the project process of the additional chip above has reached the interruptible node, the project process is interrupted and paused at this time and the current project process is continued at a later time point, which does not affect the project result of the above additional chip. However, the project process of the chip is normally not interrupted and therefore does not involve later resuming. Therefore, in order to fit the present disclosure, the applicant introduces a safe mode. The safe mode refers to a mode in which the current project process is paused, allowing the chip to enter this mode, and the current project process is continued at a later time point without affecting the current project result. It can be seen that, in general, after the project process of the chip reaches the interruptible node, the chip is controlled to enter the safe mode, such that the project process of the chip is interrupted at the current node in one aspect, and the current project process is continued at a later time point in another aspect.

Thus, in some embodiments, the state of the chip in the safe mode is an interference-permissible state; in some embodiments, after the chip is in the safe mode for more than a time threshold value, the chip is exited from the safe mode, for example, the state of the chip is changed from an interference-permissible state to an interference-impermissible state, and the interrupted project process of the chip is resumed.

In some embodiments, the time threshold value may be set in consideration of one or both of the following: the influence degree (contribution degree) of the next step on the project detection result; and the approximate time required to load or unload one or more chips. For example, for reactions that conventionally require 2 hours to complete ("conventional" means that no suspending/interruption step is intentionally performed), the time threshold value typically does not exceed 2 hours, and further, may not exceed 1 hour, 30 minutes, or 15 minutes, etc.; for another example, an appropriate time threshold value may be set in combination with the time typically required to complete the loading/unloading of a chip, for example, the time threshold value is set by 1.5, 2, 3, or 5 times the time typically required to complete the loading or unloading of a chip after reaching an interference-permissible state. In this way, when the time threshold value is reached (whether the target action is completed or not), the next step is continued, so as to improve the operation efficiency.

In some embodiments, after the chip enters the safe mode, timing is also started, a timeout prompt interface is provided if an accumulated time exceeds the set threshold value and the chip is still in the safe mode, and the timing is restarted after a confirmation instruction is acquired.

In some scenarios, a user needs to load a current chip, and if the user forgets to place the above current chip in the carrying module 51 during the operation of the sequencing system, the above additional chip is in the safe mode for a long time, which may cause inefficiency in the sequencing process of the above additional chip. Therefore, after the above additional chip enters the safe mode, timing is started; if the timing duration exceeds the set threshold value and the above additional chip is still in the safe mode, the time for the above additional chip to enter the safe mode is determined to be too long, and a timeout prompt interface is provided; and the timing is restarted after a user confirmation instruction is received. In some embodiments, the set threshold value may be set according to the actual use time of the project, and may be set to any value greater than 0, for example, 20 minutes, 30 minutes, 1 hour, or the like, which is not limited in the present application. In some embodiments, the timeout prompt interface is configured to prompt that the current entry into the safe mode has timed out; in some embodiments, the timeout prompt interface may be displayed in a jump or pop-up window form. In some embodiments, the timeout prompt interface may include a safe mode timeout prompt box, and the safe mode timeout prompt box includes a determination key. The timeout prompt interface may be closed by a selection instruction received at the determination key in the timeout prompt interface, thereby displaying an interface before the timeout prompt interface, and returning to execute the start of timing until the additional chip exits from the safe mode. The selection instruction may be the click operation of the determination key.

In some embodiments, entering the safe mode further includes adjusting the temperature of the additional chip to room temperature, and exiting from the safe mode further includes restoring the temperature of the additional chip to a temperature before the interruption. When the additional chip (or the project process of the additional chip) enters the safe mode, that is, when the additional chip (or the project process of the additional chip) is paused, the temperature of the additional chip needs to be adjusted to room temperature; when the additional chip exits from the safe mode, the temperature of the additional chip needs to be restored to the temperature before entering the safe mode, that is, the temperature before the interruption.

In some embodiments, step 104 may include steps 1041-1043 below: step 1041: determining whether the loading of the current chip has not been ended and the project process of the additional chip has reached the interruptible node. If yes, step 1042 is continued; if no, step 1043 is continued. Step 1042: ending the monitoring of the additional chip and enabling the additional chip to enter the safe mode.

If the current chip is still loaded and the interruptible node of the additional chip that has been reached is monitored, the additional chip may enter the safe mode, that is, the project process of the additional chip is interrupted, and the monitoring of the additional chip is ended. Step 105 is continued.

Step 1043: ending the monitoring of the additional chip, or exiting the additional chip from the safe mode. Step 105: upon determining that the loading of the current chip has been ended, starting execution of the project requested by the current chip, and exiting the additional chip from the safe mode. In some embodiments, exiting from the safe mode includes resuming the interrupted project process of the additional chip.

After the loading of the current chip is ended, the project of the current chip may be started, and the project process of the additional chip is started to be executed again, such that the current chip and the additional chip may run together.

Based on steps 101 to 105 described above, it can be seen that when the sequencing system needs to load the current chip, it is detected that the additional chip is executing a project, and then when the additional chip is in an interference-permissible state and there is an unreached interruptible node, the execution of the project process of the additional chip is monitored; when the loading of the current chip has not been ended, it is monitored that the additional chip has reached the interruptible node, such that the additional chip enters a safe mode until the loading of the current chip is ended; and the current chip executes the requested project, and the additional chip continues execution of the interrupted project. By setting the interruptible node, when the project process of the additional chip has an interruptible node that has not been reached yet, the project process of the additional chip is monitored, such that the project process of the additional chip is paused when the additional chip has reached the interruptible node, and when the loading of the current chip is completed, the respective projects are executed together, saving the resources. In addition, the additional chip that is executing the project enters the safe mode at an appropriate time, and other chips can be loaded in a flexible manner to run together, such that rolling on-machine operation of multiple chips is achieved, improving the overall operation efficiency of the sequencing system.

By adopting the method for chip loading according to any one of the embodiments of the present application, the sequencing system may realize the modes of loading at different times, co-operation, sequential unloading, and the like of multiple chips under the condition of original hardware configuration, thus meeting the requirements for various sequencing detection applications, enhancing the flexibility of the sequencing system, and enabling the sequencing system to have stronger industrial practicability.

In some embodiments, step 1023 may further include one or both of steps 201 and 202 below.

Step 201: upon determining the presence of an additional chip that is executing a project and that the project executed by the additional chip does not belong to the preset category, starting the loading of the current chip, and upon determining that the loading of the current chip has been ended, starting the execution of the project requested by the current chip.

When the current chip requests loading, the additional chip that is executing the project has already existed, but the project executed by the additional chip does not belong to the preset category, that is, the project executed by the additional chip may be interrupted at any time, and the operation of loading the current chip has no effect on the project executed by the additional chip. **In** this case, the loading of the current chip may be started regardless of the execution of the additional chip, and upon determining that the loading of the current chip has been ended, the execution of the project requested by the current chip is started.

Step 202: upon determining the absence of an additional chip that is executing a project, starting the loading of the current chip, and upon determining that the loading of the current chip has been ended, starting the execution of the project requested by the current chip.

When the current chip requests loading and the sequencing system does not have an additional chip that is executing a project, it may be determined that the process such as loading of the current chip and the like does not affect the projects executed by other chips, and the current chip may be directly loaded; and upon determining that the loading of the current chip has been ended, the execution of the project requested by the current chip is started.

It can be understood that, in step 102, it may be determined whether an additional chip that is executing a project is present, and then it may be determined whether the project executed by the additional chip belongs to a preset category.

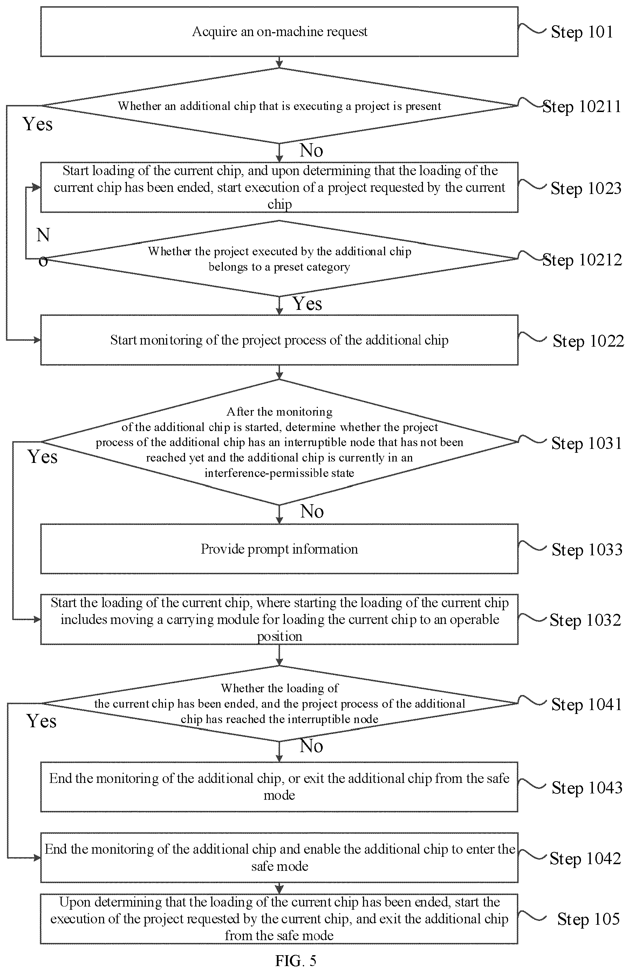

Referring to FIG. 5, FIG. 5 is a schematic flow chart of a method for controlling chip loading in some embodiments, and in FIG. 5, step 1021 may further include the following steps based on the embodiment shown in FIG. 4:
Step 10211: determining whether an additional chip that is executing a project is present. If yes, step 10212 is executed. If no, step 1023 is executed.
Step 10212: determining whether the project executed by the additional chip belongs to a preset category. If yes, step 1022 is executed. If no, step 1023 is executed.

It can be seen that, in the case that the additional chip that is executing the project is not present, and/or in the case that the project being executed by the additional chip does not belong to the preset category, the loading of the current chip may be directly started, and the project requested by the current chip is executed. Therefore, upon determining that other chips are not affected, the current chip is directly loaded and the project is executed, such that the current chip may be quickly loaded on the machine, and the two chips may execute their respective requested projects together, improving the on-machine efficiency of the chip. Moreover, the operation modes are various and flexible, meeting the requirements of different applications.

In some embodiments, step 1033 may further include one or both of steps 301 and 302 below.

Step 301: upon determining that the project process of the additional chip has the interruptible node that has not been reached yet and the additional chip is currently in an interference-impermissible state, providing prompt information including an instruction indicating that the current chip needs to wait, and starting the loading of the current chip after the additional chip is converted to be in the interference-permissible state. Step 104 is continued.

When the additional chip is in the interference-impermissible state, the current chip cannot be loaded, and after waiting for the additional chip to be in the interference-permissible state, the loading of the current chip is executed. If the project process of the additional chip has the interruptible node that has not been reached yet, step 104 may be performed. If the project process of the additional chip does not have the interruptible node that has not been reached yet, step 104 may be performed.

Step 302: upon determining that the project process of the additional chip does not have the interruptible node that has not been reached yet, providing prompt information including an instruction indicating that the current chip needs to wait, ending the monitoring of the additional chip after the additional chip is in the interference-permissible state, starting the loading of the current chip, and upon determining that the loading of the current chip has been ended, starting the execution of the project requested by the current chip.

If the additional chip is currently in the interference-impermissible state and the interference-impermissible state does not have the interruptible node, prompt information is then provided and monitoring of the state of the additional chip is started. After the additional chip is in the interference-permissible state, the monitoring of the additional chip is ended, the loading of the current chip is started, and the requested project is executed. It can be understood that, in step 103, it may be determined whether the project process of the additional chip has the interruptible node that has not been reached yet, and then it may be determined whether the additional chip is currently in the interference-permissible state; or it may be determined whether the additional chip is currently in the interference-permissible state, and then it may be determined whether the project process of the additional chip has the interruptible node that has not been reached yet; it may also be determined whether the project process of the additional chip has the interruptible node that has not been reached yet and whether the additional chip is currently in the interference-permissible state at the same time. The determination order is not limited in the present application. A specific implementation of step 103 is described in detail below with reference to the embodiment shown in FIG. 4.

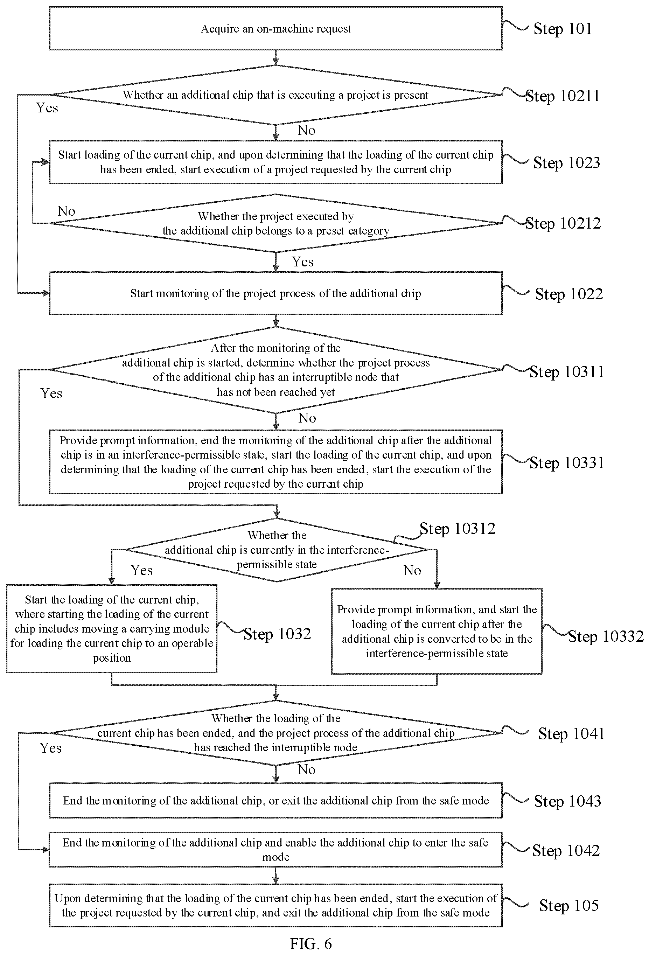

Referring to FIG. 6, FIG. 6 is a schematic flow chart of a method for controlling chip loading according to some embodiments, and in FIG. 6, step 1031 may further include the following steps based on the embodiment shown in FIG. 4 or FIG. 5:
Step 10311: after the monitoring of the additional chip is started, determining whether the project process of the additional chip has an interruptible node that has not been reached yet.

If yes, step 10312 is executed. If no, step 10331 is executed.

Step 10331: providing prompt information, ending the monitoring of the additional chip after the additional chip is in the interference-permissible state, starting the loading of the current chip, and upon determining that the loading of the current chip has been ended, starting the execution of the project requested by the current chip.

In some embodiments, the prompt information includes an instruction indicating that the current chip needs to wait. In some embodiments, step 10331 is equivalent to step 302 described above.

Step 10312: determining whether the additional chip is currently in the interference-permissible state. If yes, step 1032 is executed; if no, step 10332 is executed.

Step 10332: providing prompt information, and starting the loading of the current chip after the additional chip is converted to be in the interference-permissible state. Step 104 is continued.

For the case that one carrying module 51 corresponds to multiple chips, that is, one carrying module is shared by multiple chips, it can be understood that the contents of step 103 and step 104 may be implemented in one step or one instruction. In other words, the execution of step 103 and step 104 has no significant order or chronological order. For example, when the process of starting the loading of the current chip or loading the current chip includes moving the carrying module to an operable position, or during this process, the monitoring of the project process of the additional chip may be ended and/or the additional chip may enter the safe mode.

In some embodiments, the prompt information including an instruction indicating that the current chip needs to wait is provided. In some embodiments, step 10332 is equivalent to step 301 described above.

It can be seen that upon determining that the additional chip is in the interference-impermissible state or the project process of the additional chip does not have the interruptible node that has not been reached yet, the current chip cannot be loaded immediately, and the sequencing system provides prompt information to indicate that the current chip needs to wait; and when the loading condition is met, the current chip is loaded, such that the loading of the current chip does not interfere with the additional chip, achieving sequential loading of the chips. Moreover, the two chips may execute their respective requested projects together, improving the sequencing efficiency.

In some embodiments, step 1043 may include one, two, or three of steps 401, 402, and 403 below.

Step 401: upon determining that the loading of the current chip has been ended and the project process of the additional chip has not reached the interruptible node yet, ending the monitoring of the additional chip and starting the execution of the project requested by the current chip.

If the project process of the additional chip has not reached the interruptible node after the loading of the current chip is ended, the additional chip does not need to enter the safe mode, the execution of the project requested by the current chip may be started, and the monitoring of the additional chip is ended.

Therefore, sequential loading of the two chips is achieved, and the two chips may execute their respective requested projects together, improving the sequencing efficiency.

Step 402: upon determining that the project requested by the current chip has been canceled and the project process of the additional chip has not reached the interruptible node yet, ending the monitoring of the additional chip. If the project requested by the current chip is canceled during the monitoring of the additional chip, the monitoring of the additional chip is ended.

In some embodiments, there are multiple implementations for canceling the project requested by the current chip. For example, the implementation may be a canceling instruction received on a touch screen of the sequencing system for the project requested by the current chip, or a canceling instruction received through a canceling button on the sequencing system.

Step 403: upon determining that the project requested by the current chip has been canceled and the additional chip has entered the safe mode, exiting the additional chip from the safe mode. If the additional chip has entered the safe mode and the project requested by the current chip has been canceled, the additional chip exits the safe mode.

It can be seen that if the current chip is canceled during loading, the project process of the current chip is ended, the monitoring of the additional chip is ended or the additional chip exits the safe mode, and the additional chip continues to execute the requested project, so as to ensure the execution of the project requested by the additional chip.

In some embodiments, determining that the loading of the current chip has been ended includes acquiring a starting request of the current chip. In some embodiments, the starting request is used to start the execution of the project requested by the current chip, and may be, for example, a user-input starting request acquired via a touch screen.

In some embodiments, starting the loading of the current chip further includes providing an interaction interface for the project requested by the current chip. In some embodiments, the interaction interface may be displayed via a touch screen to display the state of the current chip and receive user instructions.

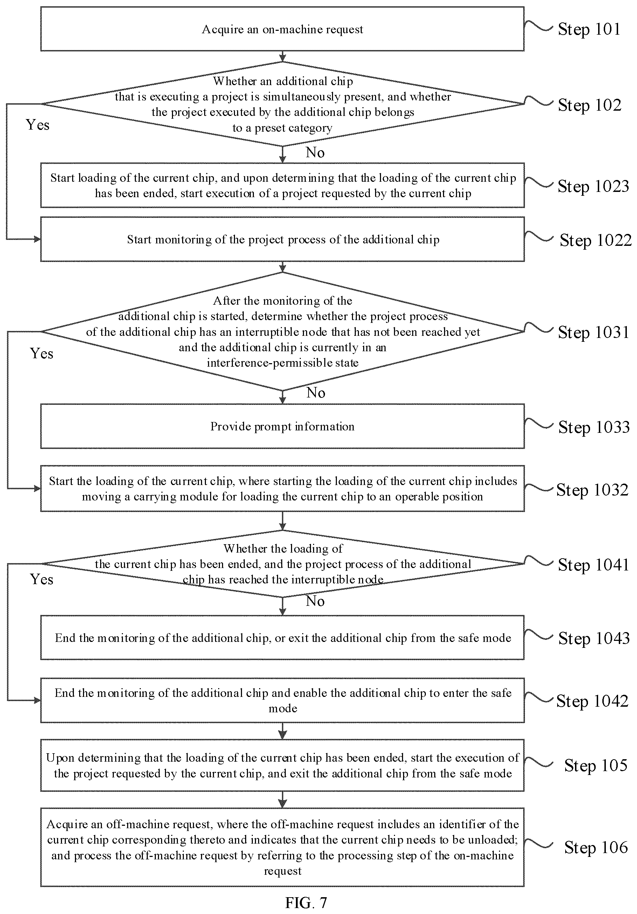

In some embodiments, projects of multiple chips have been executed in the sequencing system, and when one of the chips completes its project, it needs to perform an off-machine operation, which is similar to the above on-machine operation. At this time, a sequential off-machine operation is needed to be performed according to the current states of other chips, so as to avoid the off-machine operation from affecting other chips. The off-machine process is described in detail below with reference to the embodiment shown in FIG. 7.

Referring to FIG. 7, FIG. 7 is a schematic flow chart of a method for controlling chip loading according to some embodiments, which involves subsequent management (e.g., off-machine operation of the chip) after the chip is loaded on the machine. In some embodiments, step 105 may be followed by step 106: acquiring an off-machine request, where the off-machine request includes an identifier of the current chip corresponding thereto and indicates that the current chip needs to be unloaded; processing the off-machine request by referring to the processing step of the on-machine request, where the loading operation is replaced by the unloading operation; starting the unloading of the current chip, where starting the unloading of the current chip includes moving the carrying module loaded with the current chip to the operable position; and upon determining that the unloading of the current chip has been ended, ending the process of the current chip.

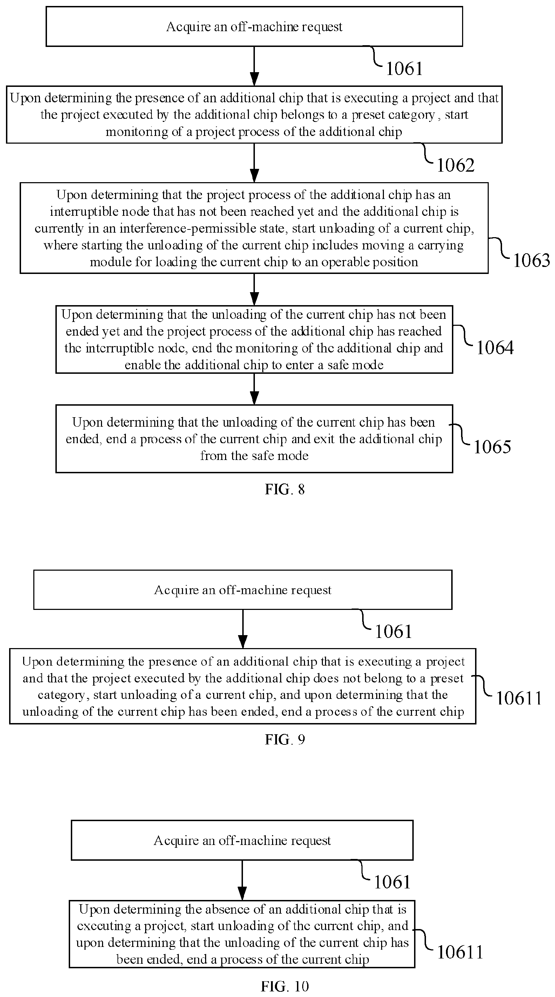

In some embodiments, referring to FIG. 8, step 106 includes the following steps: step 1061: acquiring an off-machine request, where the off-machine request includes the identifier of the current chip corresponding thereto and indicates that the current chip needs to be unloaded. Step 1062: upon determining the presence of an additional chip that is executing a project and that the project executed by the additional chip belongs to a preset category, starting monitoring of a project process of the additional chip. Step 1063: upon determining that the project process of the additional chip has an interruptible node that has not been reached yet and the additional chip is currently in an interference-permissible state, starting unloading of the current chip, where starting the unloading of the current chip includes moving a carrying module for loading the current chip to an operable position. Step 1064: upon determining that the unloading of the current chip has not been ended yet and the project process of the additional chip has reached the interruptible node, ending the monitoring of the additional chip and enabling the additional chip to enter a safe mode. Step 1065: upon determining that the unloading of the current chip has been ended, ending the process of the current chip and exiting the additional chip from the safe mode.

In some embodiments, referring to FIG. 9 or 10, after step 1061, one or both of steps 10611 and 10612 may be further included.

Step 10611: upon determining the presence of an additional chip that is executing a project and that the project executed by the additional chip does not belong to the preset category, starting the unloading of the current chip, and upon determining that the unloading of the current chip has been ended, ending the process of the current chip.

Step 10612: upon determining the absence of an additional chip that is executing a project, starting the unloading of the current chip, and upon determining that the unloading of the current chip has been ended, ending the process of the current chip.

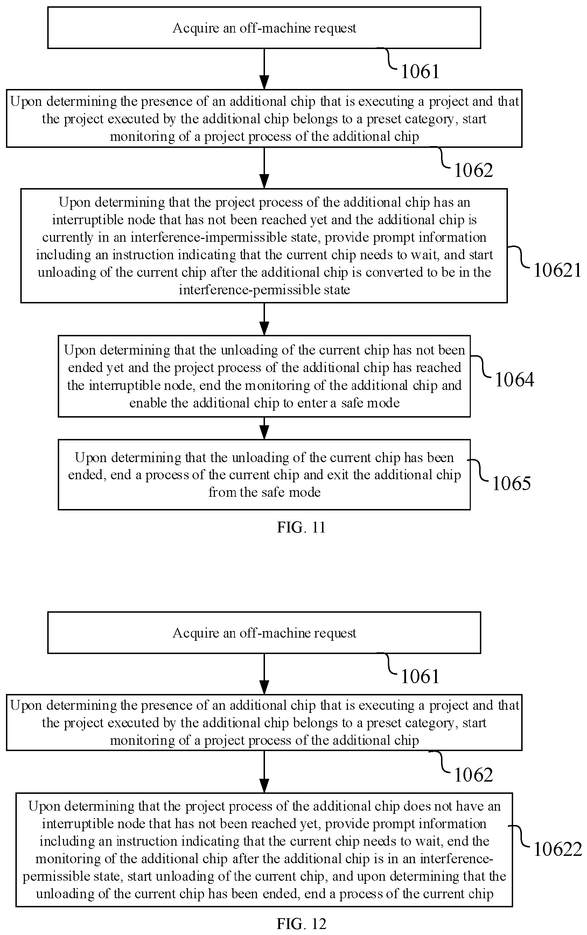

In some embodiments, referring to FIG. 11 or 12, after step 1062, one or both of steps 10621 and 10622 may be further included.

Step 10621: upon determining that the project process of the additional chip has the interruptible node that has not been reached yet and the additional chip is currently in an interference-impermissible state, providing prompt information including an instruction indicating that the current chip needs to wait, starting the unloading of the current chip after the additional chip is converted to be in the interference-permissible state, and continuing to execute step 1064.

Step 10622: upon determining that the project process of the additional chip does not have the interruptible node that has not been reached yet, providing prompt information including an instruction indicating that the current chip needs to wait, ending the monitoring of the additional chip after the additional chip is in the interference-permissible state, starting the unloading of the current chip, and upon determining that the unloading of the current chip has been ended, ending the process of the current chip.

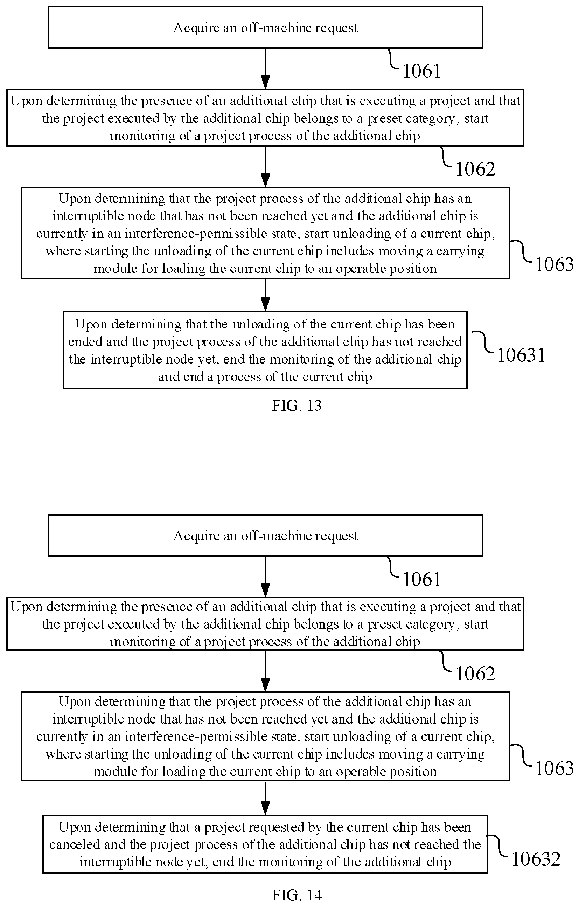

In some embodiments, the prompt information further includes a waiting time required for the current chip (determined based on the time required for the additional chip to convert to the interference-permissible state). In some embodiments, referring to FIG. 13, 14, or 15, after step 1063, one, two, or three of steps 10631, 10632, and 10633 are further included.

Step 10631: upon determining that the unloading of the current chip has been ended and the project process of the additional chip has not reached the interruptible node yet, ending the monitoring of the additional chip and ending the process of the current chip.

Step 10632: upon determining that the project requested by the current chip has been canceled and the project process of the additional chip has not reached the interruptible node yet, ending the monitoring of the additional chip.

Step 10633: upon determining that the project requested by the current chip has been canceled and the additional chip has entered the safe mode, exiting the additional chip from the safe mode.

In some embodiments, starting the unloading of the current chip further includes providing an interaction interface for the unloading of the current chip.

The method provided in the above embodiments is described below by taking chip 1 as an example to be sequenced, and it can be understood that this embodiment is only a specific example to illustrate the above embodiments, and it does not limit the above embodiments. Assuming that the sequencing system apparatus provided in this embodiment may sequence two chips at the same time, the method of the embodiment of the present application may be implemented by performing the following operations:
step a: the sequencing system receives an on-machine request of chip 1 via a display 54. The display 54 of the sequencing system may display two pages in the left and right regions, respectively, where the two pages are human-computer interaction pages corresponding to the two chips. The two pages do not affect each other.

A user may control the page corresponding to the current idle chip position via the display 54, where the experimental information (i.e., the project process) is imported into the loading interface, and then "next" on the page is clicked. If the imported experimental information is experimental information of the sequencing project, the project to be performed by chip 1 is the sequencing project, where step a corresponds to step 101 above.

Step b: the sequencing system determines whether an additional chip that is executing a project is present. It is assumed that chip 2 that is executing a project is present, where step b corresponds to step 10211 above. Step c: the sequencing system determines whether the project executed by chip 2 belongs to a preset category. It is assumed that chip 2 is executing the sequencing project, which is in the current cycle of the 1st biochemical reaction, where step c corresponds to step 10212 above.

Step d: the sequencing system starts monitoring of the project process of chip 2, where step d corresponds to step 1022 above.

Step e: the sequencing system determines whether chip 2 is currently in an interference-permissible state. If chip 2 is currently in a biochemical reaction stage and does not reach the photographing stage of this cycle, chip 1 may currently be loaded.

Step f: the sequencing system determines whether the project process of chip 2 has an interruptible node that has not been reached yet. If the user does not set the interruptible node in chip 2 before sequencing, the sequencing system defaults to setting the interruptible node before photographing. Thus, chip 2 has not reached the interruptible node before photographing, where steps e and f correspond to step 1031 above.

Step g: the sequencing system starts the loading of chip 1. The sequencing system moves the carrying module for loading chip 1 to an operable position, and the user may put chip 1 into the carrying module, such that the sequencing system performs the subsequent loading operation, where step g corresponds to step 1032 above. Step h: during the loading of chip 1, the sequencing system continuously monitors whether chip 2 reaches the interruptible node. It is assumed that during the loading of chip 1, i.e., when the loading of chip 1 has not been completed, chip 2 is monitored to complete the biochemical reaction of this cycle and is about to enter the photographing stage, where step h corresponds to step 1041.

Step i: the sequencing system ends the monitoring of chip 2 and enables chip 2 to enter a safe mode. The sequencing system ends the monitoring of chip 2 and pauses the project process of chip 2, thereby enabling chip 2 to enter the safe mode, where step i corresponds to step 1042 above.

Step j: the sequencing system continuously detects whether the loading of chip 1 is ended. If it is detected that the loading of chip 1 has been completed, the sequencing project of chip 1 is started, chip 2 is exited from the safe mode, and chip 2 may continue to execute the corresponding project, where step j corresponds to step 105 above.

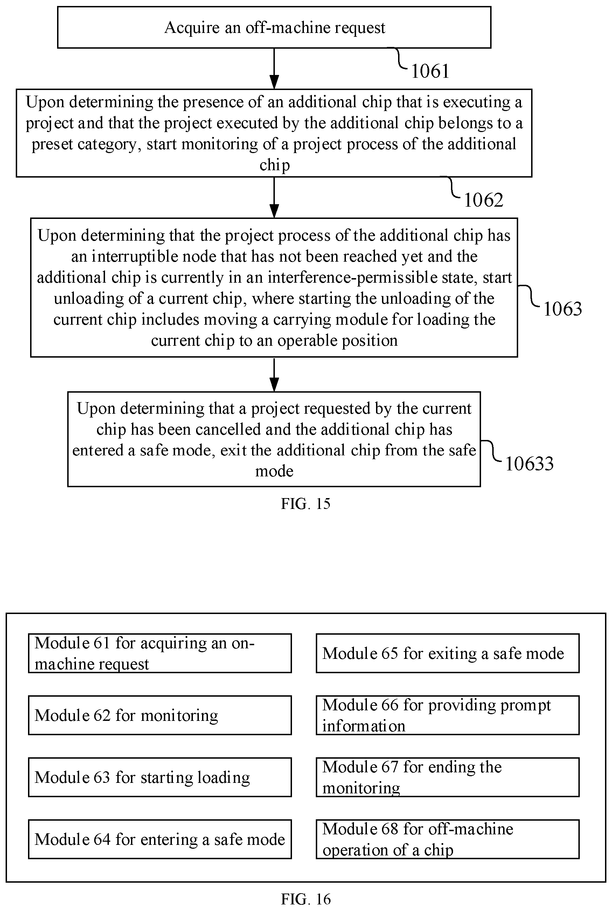

The embodiments of the present disclosure also provide an apparatus for controlling chip loading. Referring to FIG. 16, the apparatus for controlling chip loading according to some embodiments includes a module 61 for acquiring an on-machine request, a module 62 for monitoring, a module 63 for starting loading, a module 64 for entering a safe mode, and a module 65 for exiting the safe mode; in some embodiments, the apparatus for controlling chip loading further includes one or more of a module 66 for providing prompt information, a module 67 for ending the monitoring, and a module 68 for off-machine operation of a chip.

The module 61 is configured to acquire an on-machine request, and the on-machine request includes an identifier of a current chip corresponding thereto and an identifier of a project requested to be executed on a machine.

The module 62 for monitoring is configured to, upon determining the presence of an additional chip that is executing a project and that the project executed by the additional chip belongs to a preset category, start monitoring of a project process of the additional chip.

The module 63 for starting loading is configured to, after the monitoring of the additional chip is started, start loading of the current chip upon determining that the project process of the additional chip has an interruptible node that has not been reached yet and the additional chip is currently in an interference-permissible state, where starting the loading of the current chip includes moving a carrying module for loading the current chip to an operable position.

In some embodiments, the module 63 for starting loading is further configured to, upon determining the presence of an additional chip that is executing a project and that the project executed by the additional chip does not belong to the preset category, start the loading of the current chip, and upon determining that the loading of the current chip has been ended, start the execution of the project requested by the current chip.

In some embodiments, the module 63 for starting loading is further configured to, upon determining the absence of an additional chip that is executing a project, start the loading of the current chip, and upon determining that the loading of the current chip has been ended, start the execution of the project requested by the current chip.

The module 64 for entering a safe mode is configured to, upon determining that the loading of the current chip has not been ended yet and the project process of the additional chip has reached the interruptible node, end the monitoring of the additional chip and enable the additional chip to enter the safe mode, where entering the safe mode includes interrupting the project process of the additional chip.

The module 65 for exiting the safe mode is configured to, upon determining that the loading of the current chip has been ended, start execution of the project requested by the current chip, and exit the additional chip from the safe mode, where exiting from the safe mode includes resuming the interrupted project process of the additional chip.

In some embodiments, the module 66 for providing prompt information is configured to, upon determining that the project process of the additional chip has the interruptible node that has not been reached yet and the additional chip is currently in an interference-impermissible state, provide the prompt information including an instruction indicating that the current chip needs to wait, and start the loading of the current chip after the additional chip is converted to be in the interference-permissible state.

In some embodiments, the module 66 for providing prompt information is configured to, upon determining that the project process of the additional chip does not have the interruptible node that has not been reached yet, provide prompt information including an instruction indicating that the current chip needs to wait, end the monitoring of the additional chip after the additional chip is in the interference-permissible state, start the loading of the current chip, and upon determining that the loading of the current chip has been ended, start the execution of the project requested by the current chip.

In some embodiments, the module 67 for ending the monitoring is configured to end the monitoring of the additional chip and start the execution of the project requested by the current chip upon determining that the loading of the current chip has been ended and the project process of the additional chip has not reached the interruptible node yet.

In some embodiments, the module 67 for ending the monitoring is configured to end the monitoring of the additional chip upon determining that the project requested by the current chip has been canceled and the project process of the additional chip has not reached the interruptible node yet.

In some embodiments, the module 67 for ending the monitoring is configured to exit the additional chip from the safe mode upon determining that the project requested by the current chip has been canceled and the additional chip has entered the safe mode.

The module 68 for off-machine operation of a chip is configured to acquire an off-machine request, where the off-machine request includes an identifier of the current chip corresponding thereto and indicates that the current chip needs to be unloaded; process the off-machine request by referring to the processing step of the on-machine request, where the loading operation is replaced by the unloading operation; start the unloading of the current chip, where starting the unloading of the current chip includes moving the carrying module loaded with the current chip to the operable position; and upon determining that the unloading of the current chip has been ended, end the process of the current chip.

The steps or functions executed by the above modules may also refer to the description of the method for controlling chip loading herein, which will not be recited here.

It will be understood by those skilled in the art know that, in addition to implementing the controller/processor in a form of pure computer-readable program code, the same functions can be implemented in a form of a logic gate, a switch, an application-specific integrated circuit, an editable logic controller, an embedded microcontroller, and the like by logically programming the steps. Therefore, the controller/processor may be regarded as a hardware component, and a device included in the controller/processor for implementing various functions may also be regarded as a structure in the hardware component. Alternatively, a device for implementing various functions may be regarded as both a software module for implementing the method and a structure in the hardware component.

In the specification, descriptions such as "one embodiment", "some embodiments", "one or some specific embodiments", "one or some embodiments/examples", or the like, means that a particular feature, structure, or characteristic described in reference to the embodiment or example is included in at least one embodiment or example of the present disclosure. In this specification, the schematic description of the aforementioned terms does not necessarily refer to the same embodiment or example. Moreover, the specific features, structures and other characteristics described may be combined in any one or more embodiments or examples in an appropriate manner.

The principle and embodiments of the present disclosure have been illustrated by means of specific examples, and it should be understood that the embodiments above are merely intended to help understand the present disclosure, rather than limiting the present disclosure. Variations may be made to the above specific embodiments by those of ordinary skill in the art according to the principle of the present disclosure.

## Claims

1. A method for controlling chip loading, comprising:
step 101: acquiring an on-machine request, wherein the on-machine request comprises an identifier of a current chip corresponding thereto and an identifier of a project requested to be executed on a machine;
step 102: upon determining the presence of an additional chip that is executing a project and that the project executed by the additional chip belongs to a preset category, starting monitoring of a project process of the additional chip;
step 103: upon determining that the project process of the additional chip has an interruptible node that has not been reached yet and the additional chip is currently in an interference-permissible state, starting loading of the current chip, wherein starting the loading of the current chip comprises moving a carrying module for loading the current chip to an operable position;
step 104: upon determining that the loading of the current chip has not been ended yet and the project process of the additional chip has reached the interruptible node, ending the monitoring of the additional chip and enabling the additional chip to enter a safe mode, wherein entering the safe mode comprises interrupting the project process of the additional chip; and
step 105: upon determining that the loading of the current chip has been ended, starting execution of the project requested by the current chip, and exiting the additional chip from the safe mode, wherein exiting from the safe mode comprises resuming the interrupted project process of the additional chip.

2. The method according to claim 1, further comprising, after step 101,
step 201: upon determining the presence of an additional chip that is executing a project and that the project executed by the additional chip does not belong to the preset category, starting the loading of the current chip, and upon determining that the loading of the current chip has been ended, starting the execution of the project requested by the current chip;
and/or,
step 202: upon determining the absence of an additional chip that is executing a project, starting the loading of the current chip, and upon determining that the loading of the current chip has been ended, starting the execution of the project requested by the current chip.

3. The method according to claim 1, further comprising, after step 102,
step 301: upon determining that the project process of the additional chip has the interruptible node that has not been reached yet and the additional chip is currently in an interference-impermissible state, providing prompt information comprising an instruction indicating that the current chip needs to wait, starting the loading of the current chip after the additional chip is converted to be in the interference-permissible state, and continuing to execute step 104;
and/or,
step 302: upon determining that the project process of the additional chip does not have the interruptible node that has not been reached yet, providing prompt information comprising an instruction indicating that the current chip needs to wait, ending the monitoring of the additional chip after the additional chip is in the interference-permissible state, starting the loading of the current chip, and upon determining that the loading of the current chip has been ended, starting the execution of the project requested by the current chip.

4. The method according to claim 3, wherein the prompt information further comprises a waiting time required for the current chip.

5. The method according to claim 1, further comprising, after step 103,
step 401: upon determining that the loading of the current chip has been ended and the project process of the additional chip has not reached the interruptible node yet, ending the monitoring of the additional chip and starting the execution of the project requested by the current chip; and/or,
step 402: upon determining that the project requested by the current chip has been canceled and the project process of the additional chip has not reached the interruptible node yet, ending the monitoring of the additional chip;
and/or,
step 403: upon determining that the project requested by the current chip has been canceled and the additional chip has entered the safe mode, exiting the additional chip from the safe mode.

6. The method according to any one of claims 1 to 5, comprising at least one of the following features a) to g):
a) for the project belonging to the preset category, the project process thereof has more than two interruptible nodes;
b) the interruptible node is a preset node before the interference-impermissible state, or the interruptible node is a previous node that enters the interference-impermissible state;
c) entering the safe mode further comprises adjusting the temperature of the additional chip to room temperature, and exiting from the safe mode further comprises restoring the temperature of the additional chip to a temperature before the interruption;
d) after the additional chip enters the safe mode, timing is also started, a timeout prompt interface is provided if an accumulated time exceeds a set threshold value and the additional chip is still in the safe mode, and the timing is restarted after a confirmation instruction is acquired;
e) determining that the loading of the current chip has been ended comprises acquiring a starting request of the current chip, wherein the starting request is used for starting the execution of the project requested by the current chip;
f) the project belonging to the preset category is a project having the interference-impermissible state, the interference-impermissible state comprising a photographing state; and
g) starting the loading of the current chip further comprises providing an interaction interface for the project requested by the current chip.

7. The method according to any one of claims 1 to 5, further comprising:
step 106: acquiring an off-machine request, wherein the off-machine request comprises an identifier of the current chip corresponding thereto and indicates that the current chip needs to be unloaded; starting the unloading of the current chip, wherein starting the unloading of the current chip comprises moving the carrying module loaded with the current chip to the operable position; and upon determining that the unloading of the current chip has been ended, ending the process of the current chip.

8. An apparatus for controlling chip loading, comprising:
an on-machine request module, configured to acquire an on-machine request, wherein the on-machine request comprises an identifier of a current chip corresponding thereto and an identifier of a project requested to be executed on a machine;
a monitoring module, configured to, upon determining the presence of an additional chip that is executing a project and that the project executed by the additional chip belongs to a preset category, start monitoring of a project process of the additional chip;
a loading start module, configured to, upon determining that the project process of the additional chip has an interruptible node that has not been reached yet and the additional chip is currently in an interference-permissible state, start loading of the current chip, wherein starting the loading of the current chip comprises moving a carrying module for loading the current chip to an operable position;
a safe mode module, configured to, upon determining that the loading of the current chip has not been ended yet and the project process of the additional chip has reached the interruptible node, end the monitoring of the additional chip and enable the additional chip to enter a safe mode, wherein entering the safe mode comprises interrupting the project process of the additional chip; and
an exit module, configured to, upon determining that the loading of the current chip has been ended, start execution of the project requested by the current chip, and exit the additional chip from the safe mode, wherein exiting from the safe mode comprises resuming the interrupted project process of the additional chip.

9. The apparatus according to claim 8, wherein after the on-machine request module acquires the on-machine request, the loading start module is further configured to:
upon determining the presence of an additional chip that is executing a project and that the project executed by the additional chip does not belong to the preset category, start the loading of the current chip, and upon determining that the loading of the current chip has been ended, start the execution of the project requested by the current chip;
and/or,
upon determining the absence of an additional chip that is executing a project, start the loading of the current chip, and upon determining that the loading of the current chip has been ended, start the execution of the project requested by the current chip.

10. The apparatus according to claim 8, wherein after the monitoring module starts the monitoring of the project process of the additional chip, the loading start module is further configured to:
upon determining that the project process of the additional chip has the interruptible node that has not been reached yet and the additional chip is currently in an interference-impermissible state, provide prompt information comprising an instruction indicating that the current chip needs to wait, start the loading of the current chip after the additional chip is converted to be in the interference-permissible state, and notify the safe mode module, so as to, upon determining that the loading of the current chip has not been ended yet and the project process of the additional chip has reached the interruptible node, end the monitoring of the additional chip and enable the additional chip to enter the safe mode, wherein entering the safe mode comprises interrupting the project process of the additional chip; and/or,
upon determining that the project process of the additional chip does not have the interruptible node that has not been reached yet, provide prompt information comprising an instruction indicating that the current chip needs to wait, end the monitoring of the additional chip after the additional chip is in the interference-permissible state, start the loading of the current chip, and upon determining that the loading of the current chip has been ended, start the execution of the project requested by the current chip.

11. The apparatus according to claim 10, wherein the prompt information further comprises a waiting time required for the current chip.

12. The apparatus according to claim 8, wherein after the safe mode module ends the monitoring of the additional chip and enables the additional chip to enter the safe mode upon determining that the loading of the current chip has not been ended yet and the project process of the additional chip has reached the interruptible node,
the monitoring module ends the monitoring of the additional chip and starts the execution of the project requested by the current chip upon determining that the loading of the current chip has been ended and the project process of the additional chip has not reached the interruptible node yet; and/or,
the monitoring module ends the monitoring of the additional chip upon determining that the project requested by the current chip has been canceled and the project process of the additional chip has not reached the interruptible node yet; and/or,
the exit module exits the additional chip from the safe mode upon determining that the project requested by the current chip has been canceled and the additional chip has entered the safe mode.

13. The apparatus according to any one of claims 8 to 12, comprising at least one of the following features a) to g):
a) for the project belonging to the preset category, the project process thereof has more than two interruptible nodes;
b) the interruptible node is a preset node before the interference-impermissible state, or the interruptible node is a previous node that enters the interference-impermissible state;
c) entering the safe mode further comprises adjusting the temperature of the additional chip to room temperature, and exiting from the safe mode further comprises restoring the temperature of the additional chip to a temperature before the interruption;
d) the safe mode module also starts timing after the additional chip enters the safe mode, provides a timeout prompt interface if an accumulated time exceeds a set threshold value and the additional chip is still in the safe mode, and restarts the timing after acquiring a confirmation instruction;
e) determining that the loading of the current chip has been ended comprises acquiring a starting request of the current chip, wherein the starting request is used for starting the execution of the project requested by the current chip;
f) the project belonging to the preset category is a project having the interference-impermissible state, the interference-impermissible state comprising a photographing state; and
g) starting the loading of the current chip further comprises providing an interaction interface for the project requested by the current chip.

14. The apparatus according to any one of claims 8 to 12, further comprising an off-machine module configured to acquire an off-machine request, wherein the off-machine request comprises an identifier of the current chip corresponding thereto and indicates that the current chip needs to be unloaded; start the unloading of the current chip, wherein starting the unloading of the current chip comprises moving the carrying module loaded with the current chip to the operable position; and upon determining that the unloading of the current chip has been ended, end the process of the current chip.

15. A sequencing system, comprising the apparatus according to any one of claims 8 to 14.

16. A sequencing system, comprising,
one or more memories, configured to store a program; and
one or more processors, configured to execute the program to implement the method according to any one of claims 1 to 7.

17. The sequencing system according to claim 16, further comprising,
a carrying module, configured to carry and/or move a chip;
a fluid path module, connected to the carrying module and configured to control a reagent to flow in and flow out of the chip; and
a signal acquisition module, connected to the carrying module and the processor, and configured to acquire a signal from a designated field of view of the chip.

18. A computer-readable storage medium, comprising a program, wherein executing the program can implement the method according to any one of claims 1 to 7.
